(19)

Europäisches Patentamt

European Patent Office

Office européen des brevets

(11) **EP 2 937 698 A1**

(12) **EUROPEAN PATENT APPLICATION**

(43) Date of publication:
28.10.2015 Bulletin 2015/44

(51) Int Cl.:
**G01N 33/574** (2006.01)

(21) Application number: 14305615.8

(22) Date of filing: 25.04.2014

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **ImmunID**
**38040 Grenoble (FR)**

(72) Inventors:
• **Pasqual, Nicolas**
**38500 Coublevie (FR)**

• **Manuel, Manuarii**
**38000 Grenoble (FR)**
• **Courtier, Anaïs**
**38500 Voiron (FR)**
• **Mouret, Jean-François**
**38500 Coublevie (FR)**
• **Weisbuch, Sébastien**
**38430 Saint Jean de Moirans (FR)**

(74) Representative: **Marcadé, Véronique et al**
**Cabinet Orès**
**36, rue de St Pétersbourg**
**75008 Paris (FR)**

(54) **Use of immune combinatorial diversity as a predictive marker for identifying patients likely to respond to an anti-CTLA-4 treatment**

(57)    The present invention pertains to the field of cancer therapy and provides a predictive marker for determining if a patient suffering from metastatic melanoma or another cancer is likely to be a good responder to a treatment by Ipilimumab or another drug blocking an immune checkpoint.

**EP 2 937 698 A1**

**Description**

Melanoma is a major public health problem worldwide, with an incidence doubling every 10 years. This disease, easily curable in early stages, has a bad prognosis once become metastatic. Response rate for metastatic melanoma with dacarbazine, which used to be the standard chemotherapy agent, is around 10%. Polychemotherapy approaches increase response rate, with no survival benefit. Korn *et al.* have reported in a meta-analysis of 42 phase II trials of metastatic melanoma, a median survival of 6.2 months (5.9 - 6.5 months) (Korn et al., 2008). In a multivariate analysis, some factors such as performance status, visceral metastasis, gender and brain metastasis, seem to impact the survival.

After decades of disappointing results, the recent successes obtained in clinical oncology by immunotherapies confirm the relevance of immune system stimulation in this setting. Antigen 4 of cytotoxic T lymphocytes (CTLA-4) is a negative regulator of T cell activation. An anti CTLA-4 antibody, also called Ipilimumab and marketed as Yervoy™, potentiates immune response leading to activation, proliferation of T lymphocytes and their invasion of tumour. This treatment allows a more efficient adaptive immune response against tumours. This indirect anti-cancer strategy, based on long-term immune system activation to fight the tumour, allowed for the first time to increase the life expectancy of patients with metastatic melanoma and to decrease the risk of relapse (Hodi et al., 2010).

Lymphopenia following treatment has been associated with response to Yervoy™ (Ku et al., 2010). Patients with an absolute lymphocyte count (ALC) $\geq$1000/$\mu$L after 2 Yervoy™ treatments (Week 7) had a significantly improved clinical benefit rate (51 % *vs.* 0%; P = .01) and median OS (11.9 vs 1.4 months; P < .001) compared with those with an ALC <1000/$\mu$L.

Recent and promising clinical trials with an anti PD-1 and an anti-PD-L1 molecules in metastatic melanoma, non-small-cell lung cancer, colorectal cancer, renal-cell cancer, ovarian cancer, pancreatic cancer, gastric cancer, and breast cancer also showed that suppressing immune checkpoints could induce immune cells activation, thus promoting the development of an effective and durable anti-tumour adaptive response (Brahmer et al., 2012; Hamid et al., 2013; Ribas, 2012).

Unfortunately, all clinical studies on anti-CTLA4 treatment showed antitumor efficacy in only ~20% of patients, whereas they induce serious side effects. In addition, toxicity and efficacy were not correlated in a given patient. Given the high level of deleterious impact and the cost of such treatment, one of the main issues to be addressed by health care professionals is to understand why these new treatments work only for a small subset of patients. Thus, the analysis of involved immune mechanisms and the validation of predictive biomarkers represent a real challenges to better determine, before treatment, patients likely to respond to immunotherapy. Non-responder patients could then avoid heavy side effects and be directed earlier to other therapies. In addition, this could save unnecessary costs.

Hence, there is an important need for a predictive marker of a patient's response to an immune checkpoint blockade therapy, so that only the patients who are likely to really benefit from this therapy receive these potentially dangerous and expensive drugs.

As shown in the experimental part below, the inventors have now demonstrated that the diversity of the immune repertoire of TCR and BCR is a key parameter for predicting the response of a patient suffering from metastatic melanoma to an anti-CTLA4 antibody. Indeed, they observed that patients having a poor diversity of the immune repertoire at baseline fail to respond to the treatment, while those with a good diversity are most likely to respond to the treatment.

Hence, the present invention first pertains to an *in vitro* method of predicting the response of a cancer patient to treatment with a medicament or drug blocking an immune checkpoint, comprising: (i) measuring the diversity of the immune repertoire in a biological sample from said patient, and (ii) comparing the measured diversity to a predetermined threshold. A measured diversity lower than the threshold is indicative for a cancer patient who would not respond to the treatment. Of course, as for most predictive markers and despite the good sensibility and sensitivity of the marker disclosed herein, "indicative for a patient who would not respond to the treatment" does not mean that this patient would not respond to the treatment with a 100% certainty. Rather, patients whose diversity is lower than the predetermined threshold are those who would most likely not respond to the treatment, *i.e.*, the response rate of this group of patients is lower than that of the group of patients having a diversity higher than the threshold. For these patients, the risk-benefit balance is such that it is better not to treat them with a drug blocking an immune checkpoint, to avoid unnecessary side-effects and costs. Then the patient can be orientated toward a new molecule tested in a clinical trial, or a targeted therapy, or chemotherapy.

When performing the above method, patients whose measured diversity of the immune repertoire is higher than the predetermined threshold have a higher rate of response to the treatment, resulting in enrichment of responders in that group of patients.

The above method can be performed from any biological sample enabling the determination of the diversity of the immune repertoire. Non-limitative examples of such samples include a whole blood sample, a blood clot, PBMCs, a tissue biopsy, *etc.*

According to a preferred embodiment of the present invention, TCR diversity is measured. In particular, the measured TCR diversity can include or consist of TCR combinatorial diversity. According to a particular way of conducting the method according to the invention, illustrated in the experimental part below, the diversity of the immune repertoire is measured by a multiplex PCR assay allowing the simultaneous detection of a significant number of TRBV-TRBJ rearrangements. For example, this assay can be designed so as to detect the presence or absence (and/or the level) of at least 20, preferably at least 35, at least 50, at least 100 or at least 200 TRBV-TRBJ rearrangements. Of note, the results shown in the experimental part below have been obtained by analyzing 276 different TRBV-TRBJ rearrangements covering 100% of the possible combinatorial rearrangements, but the skilled artisan can select, among these, the most informative ones, and/or use primers allowing the amplification of rearrangement representative of mono- or multi-gene families, in order to routinely perform the above-described method with a decreased complexity, for example by amplifying fragments from no more than 20 TRBV-TRBJ rearrangements.

It is important to note that in the frame of the present invention, several parameters of the immune diversity can be measured. In particular, the diversity can be expressed as a percentage of the number of rearrangements which can theoretically be observed by the technology used to measure it (called "global diversity" in the following text). For example, when the measured diversity is the combinatorial diversity and is measured by a PCR assay allowing the detection of 150 TRBV-TRBJ rearrangements if they are present in the sample, the global diversity of a sample in which 75 rearrangements are observed is 50%. Another parameter of the immune diversity can advantageously be measured when performing the method according to the present invention is the percentage of observed rearrangements which are responsible for a certain amount (X%) of contribution amongst the observed rearrangements. This parameter, called "divX", reflects the homogeneity of the rearrangements present in the sample. For example, div50 corresponds to the percentage of rearrangements necessary to reach 50% of contribution amongst observed rearrangements. DivX (wherein X is comprised between 0 and 100, preferably between 20 and 80) is a parameter of diversity allowing to characterize the level of homogeneity of an immune repertoire by focusing on the most important rearrangements in term of contribution to the whole repertoire. DivX is the percentage of rearrangements necessary to reach X% of contribution amongst present rearrangements. The lower the value of divX, the more restricted the repertoire.

Calculation of divX:

Rearrangements listed in ascending order of contribution
N first (i.e. more important in term of contribution) rearrangements are considered such as :

$$\sum n \ first \ rearrangements \geq X\% \ contribution$$

$$\mathrm{Div}\ X = n \ / \ \text{number of rearrangements detected}$$

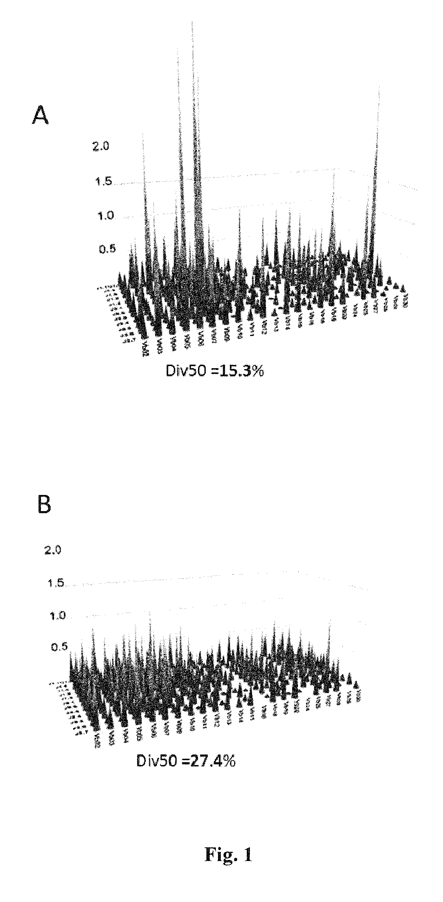

Figure 1 illustrates div50 in two different samples.

According to a particular embodiment of the present invention, the diversity of the immune repertoire is expressed as the number of rearrangements necessary to reach X% of contribution amongst the observed rearrangements (DivX), with $20 \leq X \leq 80$. For example, Div50 is measured. Of course, the skilled artisan will adapt the predetermined threshold to the parameter which is measured by assessing, in a given cohort, which couple (X, threshold) provides the best sensitivity and sensibility (ROC curves can be used to this aim).

More generally, the threshold to be considered when performing the above method is predetermined by measuring the diversity of the immune repertoire in a representative cohort of individuals treated by an immune checkpoint blockade therapy, and whose response to this treatment is known. The threshold is calculated to obtain the best predictability (sensitivity and specificity) for the response. For example, as disclosed in the experimental part below, when the combinatorial diversity of the immune repertoire at baseline (*i.e.*, before the treatment) is measured in a blood clot with a technology similar to that described in the experimental part and expressed as a percentage of observed rearrangements, a threshold of 65 to 95%, preferably 80 to 90% and for example around 85%, gives good results for predicting the response of patients suffering from metastatic melanoma to an anti-CTLA-4 antibody. In the same conditions, but if the diversity is expressed as div50, a predetermined threshold of 10 to 35%, for example around 25%, can be considered. In the same conditions, but if the diversity is expressed as div25, a predetermined threshold of 5 to 15%, for example 9.5%, can be considered. Of course, the skilled artisan is free to re-evaluate these thresholds on a larger cohort of patients, and by using any kind of technology for measuring the diversity of the immune repertoire (such as, for example, Next Generation Sequencing (NGS), spectratyping, immunoscope, Flow cytometry analysis...). The skilled artisan can also refine the threshold for particular subpopulations, depending on the type of cancer, the specific drug considered for blocking an immune checkpoint, or any other parameter regarding the patients, their pathologies (age of patient, associated treatment, type of comorbidities, ...) or their treatment (co-administration of another antineoplastic drug, different dosage regimen, *etc.).* Of course, the threshold also depends upon the parameter(s) used to assess the diversity of the immune repertoire (global diversity, Div25, Div50, *etc.)* and the skilled artisan can perfectly determine an optimal couple (parameter, threshold) or an optimal triplet (technology, parameter, threshold) to obtain a predictive method as sensitive and specific as possible.

Several new immunotherapies have been and are being developed to target the immune checkpoints. Ipilimumab (anti-CTLA4) was the first of these new therapeutics to be approved by the FDA in March 2011 for advanced melanoma, and other immunomodulators trials are ongoing in other cancers with similar encouraging results like with the anti PD-1/PD-L1. Many other immune checkpoints have been identified and could potentially be targeted. Accordingly, the present method is useful for predicting the response of a cancer patient to treatment with any medicament targeting an immune checkpoint, and in particular with a drug chosen amongst anti-CTLA-4 antibodies, anti-PD-1 antibodies and anti-PD-L1 antibodies. More particularly, it can be used for predicting the response to an anti-CTLA-4 monoclonal antibody, especially an anti-CTLA-4 human IgG1 such as Ipilimumab, or an anti-CTLA-4 human IgG2 such as Tremelimumab.

The immune system plays a dual role against cancer: it prevents tumor cell outgrowth and also sculpts the immunogenicity of the tumor cells. Drugs blocking an immune checkpoint can hence be used to treat virtually any type of cancer. Thus, the predictive method according to the invention is potentially useful for predicting a patient's response to such a drug of patients having a cancer selected amongst adrenal cortical cancer, anal cancer, bile duct cancer *(e.g.* periphilar cancer, distal bile duct cancer, intrahepatic bile duct cancer), bladder cancer, bone cancers *(e.g.* osteoblastoma, osteochrondroma, hemangioma, chondromyxoid fibroma, osteosarcoma, chondrosarcoma, fibrosarcoma, malignant fibrous histiocytoma, giant cell tumor of the bone, chordoma, lymphoma, multiple myeloma), brain and central nervous system cancers (e.g. meningioma, astocytoma, oligodendrogliomas, ependymoma, gliomas, medulloblastoma, ganglioglioma, Schwannoma, germinoma, craniopharyngioma), breast cancer *(e.g.* ductal carcinoma *in situ,* infiltrating ductal carcinoma, infiltrating lobular carcinoma, lobular carcinoma *in situ,* gynecomastia), Castleman disease *(e.g.* giant lymph node hyperplasia, angiofollicular lymph node hyperplasia), cervical cancer, colorectal cancer, endometrial cancers *(e.g.* endometrial adenocarcinoma, adenocanthoma, papillary serous adnocarcinoma, clear cell), esophagus cancer, gallbladder cancer (mucinous adenocarcinoma, small cell carcinoma), gastrointestinal carcinoid tumors (e.g. choriocarcinoma, chorioadenoma destruens), Hodgkin's disease, non-Hodgkin's lymphoma, Kaposi's sarcoma, kidney cancer (e.g. renal cell cancer), laryngeal and hypopharyngeal cancer, liver cancers *(e.g.* hemangioma, hepatic-adenoma, focal nodular hyperplasia, hepatocellular carcinoma), lung cancers (e.g. small cell lung cancer, non-small cell lung cancer), mesothelioma, plasmacytoma, nasal cavity and paranasal sinus cancer *(e.g.* esthesioneuroblastoma, midline granuloma), nasopharyngeal cancer, neuroblastoma, oral cavity and oropharyngeal cancer, ovarian cancer, pancreatic cancer, penile cancer, pituitary cancer, prostate cancer, retinoblastoma, rhabdomyosarcoma *(e.g.* embryonal rhabdomyosarcoma, alveolar rhabdomyosarcoma, pleomorphic rhabdomyosarcoma), salivary gland cancer, skin cancer *(e.g.* melanoma, nonmelanoma skin cancer), stomach cancer, testicular cancers *(e.g.* seminoma, nonseminoma germ cell cancer), thymus cancer, thyroid cancers (*e.g.* follicular carcinoma, anaplastic carcinoma, poorly differentiated carcinoma, medullary thyroid carcinoma, thyroid lymphoma), vaginal cancer, vulvar cancer, and uterine cancer *(e.g.* uterine leiomyosarcoma). More particularly, the method according to the invention can be used for predicting a patient's response to a medicament targeting an immune checkpoint, wherein the patient has a cancer selected from the group consisting of metastatic melanoma. non-small cells lung carcinoma (NSCLC), small cell lung cancer (SCLC), prostate cancer and prostatic neoplasms (especially metastatic homrmone-refractory prostate cancer), sarcoma, Wilm's tumor, lymphoma, neuroblastoma, and bladder cancer.

As recalled above, several parameters of the diversity of the immune repertoire can be measured to characterize this repertoire. In particular, the global percentage of the diversity reflects its richness, whereas DivX parameters, such as Div25 or Div50, reflect its homogeneity. According to the present invention, these two parameters can be combined to more precisely predict a patient's response. When combining two parameters like this, a patient having both measured parameters above the corresponding predetermined thresholds will be most likely a good responder to the treatment, while a patient having only one parameter above the corresponding threshold will have an intermediate probability to respond to the treatment, and a patient having both parameters below the corresponding thresholds would most likely not respond to the treatment (and hence, will normally not receive it).

According to another embodiment, the method according to the present invention is carried out by combining the measure of the diversity of the immune repertoire to the absolute lymphocyte count (ALC). In this case, an ALC superior to a predetermined threshold (typically, $1000/\mu L$), in addition to a measured diversity above the corresponding predetermined threshold, is indicative for a cancer patient who will most likely respond to the treatment.

The inventors have also demonstrated that DivX, in particular Div25, can be a prognostic marker for patients suffering from metastatic melanoma. According to another aspect, the present invention pertains to a method for *in vitro* determining if a patient suffering from metastatic melanoma has an increased risk of death, wherein a patient suffering from metastatic melanoma and having a Div25 $\leq$ 9,6% at baseline has an increased risk of death and a patient suffering from metastatic melanoma and having a Div25 > 9,6% at baseline is likely to survive longer.

According to a further aspect, the present invention pertains to a method of treating a cancer patient, comprising a first step of performing a method as described above to predict the patient's response to an immune checkpoint blockade therapy, and in case the patients is identified as a good responder to such a therapy, a second step of administering a drug blocking an immune checkpoint to the patient.

The present application also pertains to a method of treating a cancer patient identified as a good responder to an immune checkpoint blockade therapy by a method as described above, comprising administering a drug blocking an immune checkpoint to the patient.

In the above treatment methods, the drug can be selected from any immune check point blockade drug, especially the group consisting of an anti-CTLA-4 antibody, and anti-PD-1 antibody and an anti-PD-L1 antibody. In particular, it can be an anti-CTLA-4 monoclonal antibody, especially an anti-CTLA-4 monoclonal IgG1 such as Ipilimumab. According to a particular embodiment, the method is carried out by administering Ipilimumab to a responder patient at the dose of 3mg/kg every 3 weeks, for a total of 4 cures.

Of course, the same considerations as those mentioned above concerning the predicting methods also apply to the treatment methods. In particular, the patient can suffer from virtually any cancer. According to a particular aspect, the patient suffers from metastatic melanoma.

The present invention also pertains to a method of treating a cancer patient identified as a non responder to an immune checkpoint blockade therapy by a predictive method as described above, comprising administering dacarbazine to the patient.

The present invention also pertains to Ipilimumab, for use as a medicament for treating a patient suffering from metastatic melanoma and having a Div50 > 25% at baseline, and/or having a Div25 > 9.5% at baseline, and/or whose immune repertoire diversity is at least 85% at baseline.

The present invention will be understood more clearly from the further description which follows, which refers to examples illustrating the predictive value of combinatorial diversity to determine, at baseline, if a patient having a metastatic melanoma is likely to respond to a treatment by an anti-CTLA4 antibody, as well as to the appended figures.

Figure 1: In example A), 36 amongst 235 pics allow to reach 50% of the contribution (15,3% of the 235 pics). In example B), 70 amongst 255 pics allow to reach 50% of the contribution (27,4% of the 255 pics).

Figure 2: Comparison of TRB diversity levels at D0 between responders (R) and non responders (NR) patients (Wilcoxon rank sum test, p-value=0,033).

Figure 3: ROC analysis for response assessment as a function of combinatorial diversity.

Figure 4: Patient's response according to combinatorial diversity. Pearson's Chi-squared test with Yates' continuity correction (p-value= 0.147)

Figure 5: Comparison of div50 levels at D0 between responders (R) and non responders (NR) patients (Wilcoxon rank sum test, p-value=0,028).

Figure 6: ROC analysis for response assessment as a function of div50.

Figure 7: Div50 as a function of hTRB diversity.

Figure 8: Comparison of div25 levels at D0 between responders (R) and non responders (NR) patients (Wilcoxon rank sum test, p-value=0.028)

Figure 9: ROC analysis for response assessment as a function of div25

Figure 10: Patient's response according to div25. Pearson's Chi-squared test with Yates' continuity correction (p-value=0.066)

Figure 11: Analysis of Overall Survival (OS) according to baseline div25 (<9.6% or ≥9.6%) (log rank p-value = 0.0478).

Figure 12: Patient's early death according to div25 (<9.6% or ≥9.6%). Pearson's Chi-squared test with Yates' continuity correction (p-value = 0.488).

Figure 13: Absolute Lymphocyte Count (ALC) as a function of div50.

## EXAMPLES

### 1. Materials and Methods

*Response assessment*

Response was assessed by practitioner according to their expertise.

*Patient's selection*

Patients were classified into a response group according to physician expertise. Responders correspond to patients who showed particularly high clinical benefit. Non-responders correspond to patients who showed no clinical benefit at all.

Cohort characteristics:

No difference could be observed in div50<25% and div50 ≥25% arms in terms of age, LDH rate and absolute lymphocyte count (ALC) (Table 1). All the patients of that study had a Karnofsky index >90%.

|  |  | N | Min | Median | Mean | Max | Sd | C.V. | Wilcoxon test p-value |
|---|---|---|---|---|---|---|---|---|---|
| Age (year) | Div50<25% | 7 | 52 | 68 | 67,9 | 77 | 8,45 | 0,125 | 0,81 |
|  | Div50≥25% | 5 | 33 | 66 | 58,2 | 78 | 21,29 | 0,366 |  |
| LDH | Div50 <25% | 6 | 183 | 211 | 245,2 | 438 | 96,45 | 0,393 | 0,29 |
|  | Div50 ≥25% | 1 | 156 | 156 | 156 | 156 |  |  |  |
| ALC | Div50<25% | 7 | 0,4 | 1 | 1 | 1,5 | 0,37 | 0,372 | 0,06 |
|  | Div50≥25% | 5 | 1,1 | 1,5 | 1,4 | 1,9 | 0,34 | 0,239 |  |

***Combinatorial diversity analysis*** *(Multiplex PCR* **assay).**

Genomic DNA was extracted from blood clot using QIAamp DNA Blood Mini Kit (Qiagen) and concentrated using Amicon ultra 0.5 mL Centrifugal Filters (Millipore). Multiplex PCR was performed using an upstream primer specific of all functional members of a given V family and a downstream primer specific of a given J segment, as described in the patent application WO2009/095567. This assay allows the simultaneous detection of 276 different TRBV-TRBJ rearrangements covering 100% of the possible combinatorial rearrangements. All V-J1, J2, J3, J4, ..., Jn products were separated as a function of their size with a maximum amplicon size expected of ∼5 kb. PCR signals were detected and analyzed using the Constel'ID software developed by ImmunID Technologies (Grenoble, France). It is here important to mention that the multiplex PCR strategy could potentially amplify a DNA fragment corresponding to a rearrangement involving a Vx+1 gene located 3' of the targeted Vx gene. However, these side products were recognized and excluded from the final results by the Constel'ID software.

PCR products were generated using iProof GC rich Master Mix (Bio-Rad) with cycling condition as follows: 98°C for 3 min, 98°C for 20 sec, 72°C for 20 sec and 72°C for 3 min 30 sec and reducing the annealing temperature by one degree every cycle until 68°C; last cycle at 68°C; final temperature was then repeated 27 times. Finally, one cycle of 3 min at 72°C was performed. In order to normalize DNA quantity in each reaction, the actin gene was amplified in the same PCR run. PCR products were purified by ultrafiltration (Nucleofast 96, Macherey-Nalgen) and separated on Lab on chip (HT DNA 12K LabChip Kit), by microfluidic migration (Labchip GX, PERKIN ELMER). Constel'ID software was used to analyse all the data provided by the migration.

***Statistical analysis***

Results are presented as box plots with individual values. Comparisons between groups were made using the nonparametric Mann-Whitney U test (response *vs.* non response). The nonparametric Wilcoxon's paired test was used to assess variations between time points. Fisher's exact test or Yates'Chi2 test was used to compare proportions. Spearman's correlation test was used to assess correlations between TCR diversity values and clinical and biological variables. A receiver operating characteristic curve (ROC) was performed to determine the cutoff values for TCR diversity and div50 with regard to prediction of response to Yervoy. The best cutoff value was selected based on likelihood ratio and Youden's index. Statistical analyses were performed using the free statistical software package R. A p value < 0.05 was considered statistically significant.

## 2. <u>Predictive value of the combinatorial TCR diversity</u>

In a recent publication (CTLA4 Blockade Broadens the Peripheral T-Cell Receptor Repertoire. Robert L Clin Cancer Res. 2014 Apr 9) the analysis of the immune repertoire at the CDR3 level (NGS) was not able to predict response to anti-CTLA4 patient.

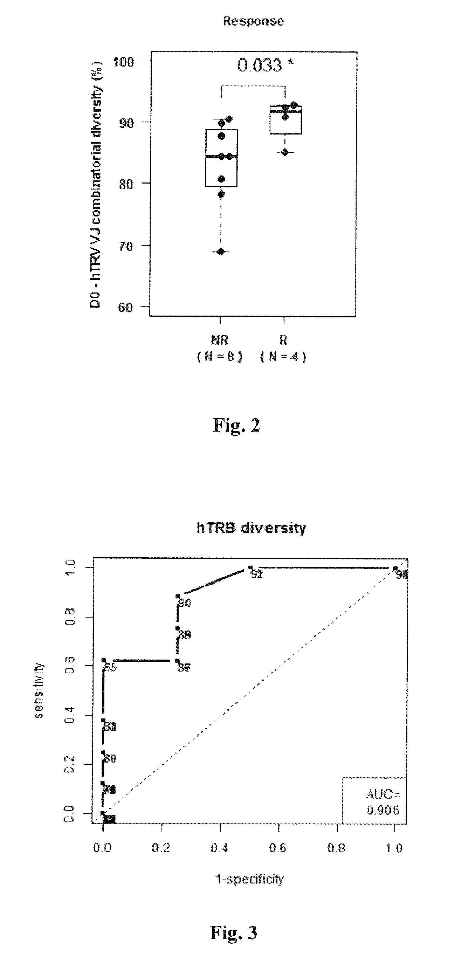

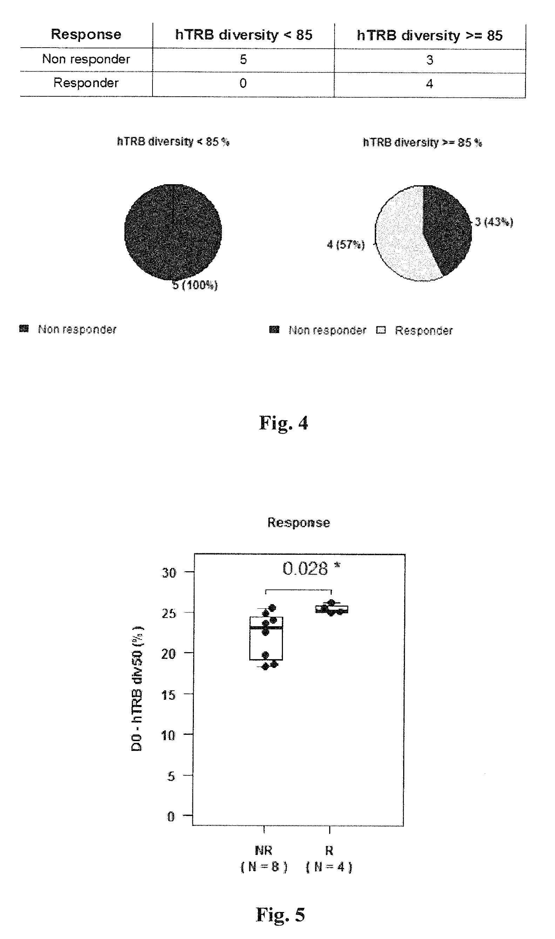

In a case study, the inventors now compared combinatorial diversity of 8 non responders & 4 responder patients to Yervoy™ (BMS anti-CTLA-4) immunotherapy in metastatic melanoma. Analysis performed on blood clot showed that response was associated with better diversity measured at baseline (Figure 2)(Wilcoxon rank sum test, p-value=0,033). A ROC analysis was performed and 85% diversity was chosen as threshold in order to optimize specificity (Figure 3). Surprisingly, no patient having a combinatorial diversity <85% (n=5) responded to the Yervoy™. At the opposite, 4 patients out of 7 with a diversity >85% diversity responded to Yervoy™ (Figure 4).

### 3. Predictive value of div50

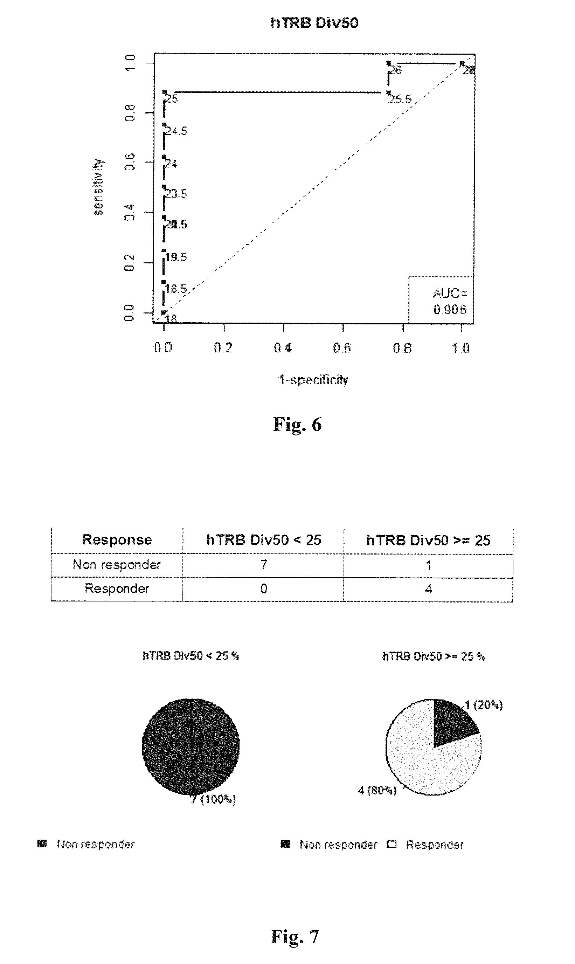

Combinatorial diversity was further assessed through div50 analysis and showed a positive correlation between response to treatment and homogeneity of the repertoire at baseline. Indeed, responder patients had a significantly higher div50 compared to non-responders (Figure 5)(Wilcoxon rank sum test, p-value=0,028). A ROC analysis was performed and 25% div50 was chosen as threshold (Figure 6). No patient having restricted repertoire, *i.e.*, div50 <25% (n=7) responded to the Yervoy™. To the contrary, 4 patients out of 5 with a div50 >25% responded to Yervoy™ (Figure 7)(Pearson's Chi-squared test with Yates' continuity correction (p-value= 0.023).

These results show that a diversified immune lymphocyte repertoire measured in peripheral blood is necessary in order to respond to Yervoy™ immunotherapy in metastatic melanoma.

### 4. Predictive value of div25

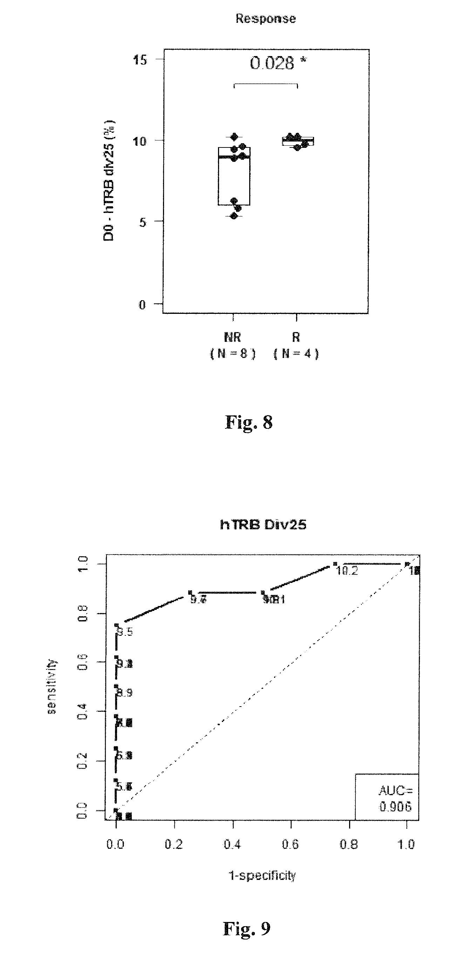

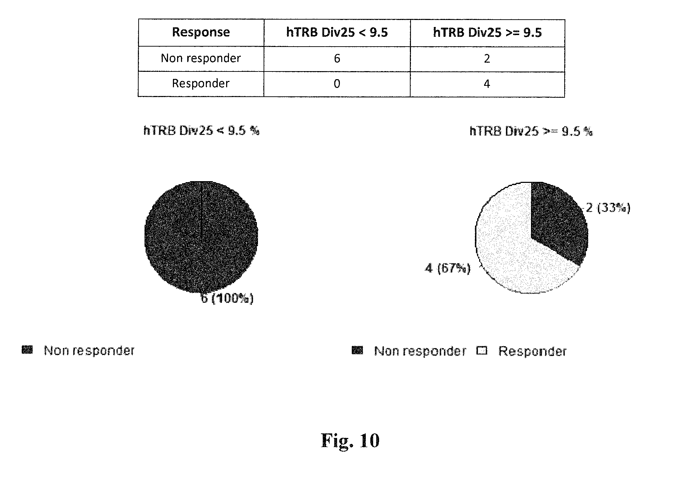

Combinatorial diversity was also assessed through div25 analysis and showed a positive correlation between response to treatment and homogeneity of the repertoire at baseline. Indeed, responder patients had a significantly higher div25 compared to non-responders (Fig. 8)(Wilcoxon rank sum test, p-value=0,028). A ROC analysis was performed and 9.5% div25 was chosen as threshold (Fig. 9). No patient having restricted repertoire, *i.e.,* div25 <9.5% (n=6) responded to the Yervoy™. To the contrary, 4 patients out of 6 with a div25 ≥9.5% responded to Yervoy™ (Fig. 10)(Pearson's Chi-squared test with Yates' continuity correction (p-value= 0.023)).

These results show that a diversified immune lymphocyte repertoire measured in peripheral blood is necessary in order to respond to Yervoy™ immunotherapy in metastatic melanoma.

### 5. Pronostic value of div25

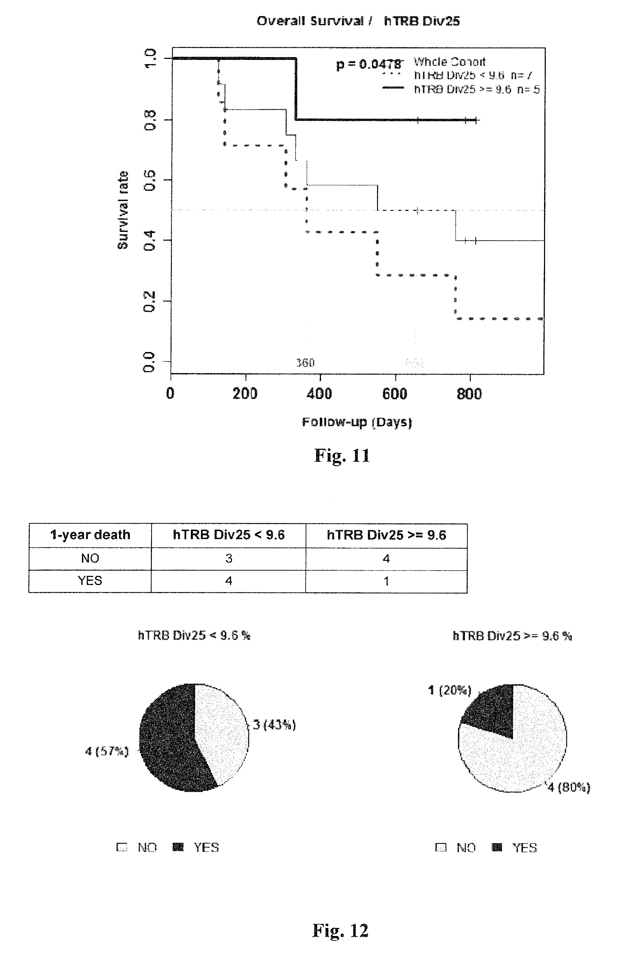

An overall survival analysis show a positive correlation between a div25 >9.6% and a better survival (Fig. 11) (log-rank p-value = 0.0478). Only 1 patient out of 5 with a good div25 *(i.e.* ≥9.6%) deceased before one year. To the contrary, 4 patients out of 7 with a low div25 *(i.e.* <9.6%) deceased before 1 year (Fig. 12).

These results pinpoint the importance of diversity analysis as a biomarker to stratify patients according to their life expectancy.

### 6. Predictive value of the combination of Div50 and global combinatorial TCR diversity

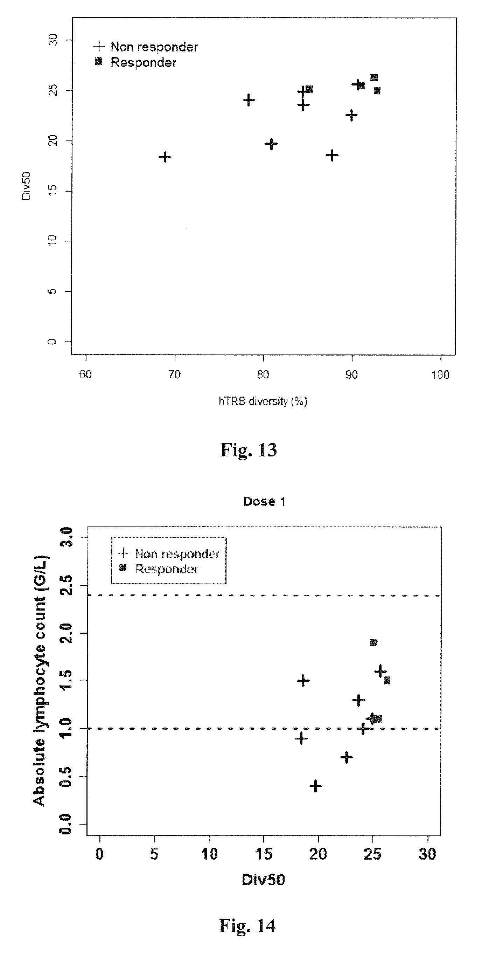

The study of Div50 as a function of diversity highlights the potential of that approach for a better stratification of responders and non-responders to Yervoy™ immunotherapy. Responders seem to have both a good div50 and diversity, as illustrated in Fig. 13. Even if Div50 stratification alone is enough in order to stratify responders in the cohort studied herein, the combination of diversity and div50 could help by giving a more accurate picture of the immune status of the patient.

### 7. Predictive value of the combination of Div50 and global Lymphocyte count (ALC)

The study of Div50 as a function of Absolute Lymphocyte Count (ALC) highlights the potential of that approach for a better stratification of responders and non-responders to Yervoy™ immunotherapy. Responders seem to have both a good div50 and ALC as illustrated in Fig. 14. Even if Div50 stratification alone is enough in order to stratify responders in the cohort studied herein, the combination of ALC and div50 could help by giving a more accurate picture of the immune status of the patient.

### REFERENCES

Brahmer, J.R., Tykodi, S.S., Chow, L.Q.M., Hwu, W.-J., Topalian, S.L., Hwu, P., Drake, C.G., Camacho, L.H., Kauh, J., Odunsi, K., et al. (2012). Safety and activity of anti-PD-L1 antibody in patients with advanced cancer. N. Engl. J. Med. 366, 2455-2465.

Galon, J., Costes, A., Sanchez-Cabo, F., Kirilovsky, A., Mlecnik, B., Lagorce-Pagès, C., Tosolini, M., Camus, M., Berger, A., Wind, P., et al. (2006). Type, density, and location of immune cells within human colorectal tumors predict clinical outcome. Science 313, 1960-1964.

Hamid, O., Robert, C., Daud, A., Hodi, F.S., Hwu, W.-J., Kefford, R., Wolchok, J.D., Hersey, P., Joseph, R.W., Weber, J.S., et al. (2013). Safety and Tumor Responses with Lambrolizumab (Anti-PD-1) in Melanoma. N. Engl. J.

Med.

Hodi, F.S., O'Day, S.J., McDermott, D.F., Weber, R.W., Sosman, J.A., Haanen, J.B., Gonzalez, R., Robert, C., Schadendorf, D., Hassel, J.C., et al. (2010). Improved Survival with Ipilimumab in Patients with Metastatic Melanoma. N. Engl. J. Med. 363, 711-723.

Korn, E.L., Liu, P.-Y., Lee, S.J., Chapman, J.-A.W., Niedzwiecki, D., Suman, V.J., Moon, J., Sondak, V.K., Atkins, M.B., Eisenhauer, E.A., et al. (2008). Meta-analysis of phase II cooperative group trials in metastatic stage IV melanoma to determine progression-free and overall survival benchmarks for future phase II trials. J. Clin. Oncol. Off. J. Am. Soc. Clin. Oncol. 26, 527-534.

Manuel, M., Tredan, O., Bachelot, T., Clapisson, G., Courtier, A., Parmentier, G., Rabeony, T., Grives, A., Perez, S., Mouret, J.-F., et al. (2012). Lymphopenia combined with low TCR diversity (divpenia) predicts poor overall survival in metastatic breast cancer patients. Oncoimmunology 1, 432-440.

Pages, F., Berger, A., Camus, M., Sanchez-Cabo, F., Costes, A., Molidor, R., Mlecnik, B., Kirilovsky, A., Nilsson, M., Damotte, D., et al. (2005). Effector memory T cells, early metastasis, and survival in colorectal cancer. N. Engl. J. Med. 353, 2654-2666.

Ribas, A. (2012). Tumor Immunotherapy Directed at PD-1. N. Engl. J. Med. 366, 2517-2519.

## Claims

1. An *in vitro* method of predicting the response of a cancer patient to treatment with a drug blocking an immune checkpoint, comprising: measuring diversity of the immune repertoire in a biological sample from said patient and comparing the measured diversity to a predetermined threshold, wherein a measured diversity lower than said threshold is indicative for a cancer patient who would not respond to the treatment, and a measured diversity higher than said threshold is indicative for a cancer patient who will respond to the treatment.

2. The method of claim 1, wherein said drug blocking an immune checkpoint is selected from the group consisting of an anti-CTLA-4 antibody, an anti-PD-1 antibody and an anti-PD-L1 antibody.

3. The method of claim 2, wherein said anti-CTLA-4 antibody is an anti-CTLA-4 monoclonal antibody.

4. The method of claim 3, wherein said anti-CTLA-4 monoclonal IgG1 is Ipilimumab.

5. The method according to any of the preceding claims, wherein said cancer patient suffers from metastatic melanoma.

6. The method according to any of the preceding claims, wherein said biological sample is selected from the group consisting of a whole blood sample, a blood clot, PBMCs and a tissue biopsy.

7. The method according to any of the preceding claims, wherein said measured diversity is TCR diversity.

8. The method according to any of the preceding claims, wherein said measured TCR diversity is combinatorial TCR diversity.

9. The method according to any of the preceding claims, wherein said measured diversity of the immune repertoire is measured by a multiplex PCR assay allowing the simultaneous detection of at least 20 TRBV-TRBJ rearrangements.

10. The method according to any of the preceding claims, wherein the diversity of the immune repertoire is expressed as the number of rearrangements necessary to reach X% of contribution amongst the observed rearrangements (DivX), wherein X is comprised between 20 and 80.

11. The method according to any of the preceding claims, wherein a patient suffering from metastatic melanoma and having a combinatorial TCR diversity $\leq$ 85% at baseline is most likely not to respond to Ipilimumab, and a patient suffering from metastatic melanoma and having a combinatorial TCR diversity > 85% at baseline is likely to respond to Ipilimumab.

12. The method according to any of the preceding claims, wherein a patient suffering from metastatic melanoma and having a Div50 $\leq$ 25% at baseline is identified as a non-responder to Ipilimumab, and a patient suffering from metastatic melanoma and having a Div50 > 25% at baseline is identified as a responder to Ipilimumab.

13. The method according to any of the preceding claims, wherein a patient suffering from metastatic melanoma and having a Div25 $\leq$ 9.5% at baseline is identified as a non-responder to Ipilimumab, and a patient suffering from metastatic melanoma and having a Div25 > 9.5% at baseline is identified as a responder to Ipilimumab.

14. The method according to any of the preceding claims, wherein the diversity of the immune repertoire is combined to the absolute lymphocyte count (ALC), and wherein an ALC superior to a predetermined threshold of 1000/$\mu$L is indicative for a cancer patient who will respond to the treatment.

15. The method according to any of the preceding claims, wherein the diversity of the immune repertoire is expressed both as DivX with X comprised between 25 and 50 and as a global percentage of the diversity, and wherein the two results are combined.

Fig. 1

Response

Fig. 2

hTRB diversity

Fig. 3

| Response | hTRB diversity < 85 | hTRB diversity >= 85 |
|---|---|---|
| Non responder | 5 | 3 |
| Responder | 0 | 4 |

hTRB diversity < 85 %

hTRB diversity >= 85 %

5 (100%)

4 (57%)   3 (43%)

▩ Non responder

▩ Non responder  ☐ Responder

**Fig. 4**

Response

0.028 *

NR
( N = 8 )   R
( N = 4 )

**Fig. 5**

### hTRB Div50

**Fig. 6**

| Response | hTRB Div50 < 25 | hTRB Div50 >= 25 |
|---|---|---|
| Non responder | 7 | 1 |
| Responder | 0 | 4 |

hTRB Div50 < 25 %      hTRB Div50 >= 25 %

7 (100%)

1 (20%)

4 (80%)

■ Non responder          ■ Non responder   □ Responder

**Fig. 7**

## Response

**Fig. 8**

## hTRB Div25

**Fig. 9**

| Response | hTRB Div25 < 9.5 | hTRB Div25 >= 9.5 |
|---|---|---|
| Non responder | 6 | 2 |
| Responder | 0 | 4 |

hTRB Div25 < 9.5 %

hTRB Div25 >= 9.5 %

6 (100%)

2 (33%)

4 (67%)

■ Non responder

■ Non responder ☐ Responder

**Fig. 10**

Overall Survival / hTRB Div25

**Fig. 11**

| 1-year death | hTRB Div25 < 9.6 | hTRB Div25 >= 9.6 |
|---|---|---|
| NO | 3 | 4 |
| YES | 4 | 1 |

hTRB Div25 < 9.6 %

hTRB Div25 >= 9.6 %

**Fig. 12**

**Fig. 13**

**Fig. 14**

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/055561 A1 (ADAPTIVE BIOTECHNOLOGIES CORP [US]; SLOAN KETTERING INST CANCER [US]) 10 April 2014 (2014-04-10) * page 15, paragraph 58; figure 6 * | 1-15 | INV. G01N33/574 |
| X | WO 2014/008448 A1 (SLOAN KETTERING INST CANCER [US]) 9 January 2014 (2014-01-09) * claims 3,10,11,12 * | 1 | |
| X,D | L. ROBERT ET AL: "CTLA4 Blockade Broadens the Peripheral T-Cell Receptor Repertoire", CLINICAL CANCER RESEARCH, vol. 20, no. 9, 28 February 2014 (2014-02-28), pages 2424-2432, XP055145637, ISSN: 1078-0432, DOI: 10.1158/1078-0432.CCR-13-2648 * abstract * | 1-7 | |
| | | | TECHNICAL FIELDS SEARCHED (IPC) |
| | | | G01N |

The present search report has been drawn up for all claims

| Place of search | Date of completion of the search | Examiner |
|---|---|---|
| Munich | 14 October 2014 | Fleitmann, J |

EPO FORM 1503 03.82 (P04C01)

**ANNEX TO THE EUROPEAN SEARCH REPORT
ON EUROPEAN PATENT APPLICATION NO.**                    EP 14 30 5615

This annex lists the patent family members relating to the patent documents cited in the above-mentioned European search report.
The members are as contained in the European Patent Office EDP file on
The European Patent Office is in no way liable for these particulars which are merely given for the purpose of information.

14-10-2014

| Patent document cited in search report | Publication date | Patent family member(s) | Publication date |
|---|---|---|---|
| WO 2014055561 A1 | 10-04-2014 | NONE | |
| WO 2014008448 A1 | 09-01-2014 | NONE | |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009095567 A

### Non-patent literature cited in the description

- **ROBERT L.** CTLA4 Blockade Broadens the Peripheral T-Cell Receptor Repertoire. *Clin Cancer Res,* 09 April 2014
- **BRAHMER, J.R. ; TYKODI, S.S. ; CHOW, L.Q.M. ; HWU, W.-J. ; TOPALIAN, S.L. ; HWU, P. ; DRAKE, C.G. ; CAMACHO, L.H. ; KAUH, J. ; ODUNSI, K. et al.** Safety and activity of anti-PD-L1 antibody in patients with advanced cancer. *N. Engl. J. Med.,* 2012, vol. 366, 2455-2465
- **GALON, J. ; COSTES, A. ; SANCHEZ-CABO, F. ; KIRILOVSKY, A. ; MLECNIK, B. ; LAGORCE-PAGÈS, C. ; TOSOLINI, M. ; CAMUS, M. ; BERGER, A. ; WIND, P. et al.** Type, density, and location of immune cells within human colorectal tumors predict clinical outcome. *Science,* 2006, vol. 313, 1960-1964
- **HAMID, O. ; ROBERT, C. ; DAUD, A. ; HODI, F.S. ; HWU, W.-J. ; KEFFORD, R. ; WOLCHOK, J.D. ; HERSEY, P. ; JOSEPH, R.W. ; WEBER, J.S. et al.** Safety and Tumor Responses with Lambrolizumab (Anti-PD-1) in Melanoma. *N. Engl. J. Med,* 2013
- **HODI, F.S. ; O'DAY, S.J. ; MCDERMOTT, D.F. ; WEBER, R.W. ; SOSMAN, J.A. ; HAANEN, J.B. ; GONZALEZ, R. ; ROBERT, C. ; SCHADENDORF, D. ; HASSEL, J.C. et al.** Improved Survival with Ipilimumab in Patients with Metastatic Melanoma. *N. Engl. J. Med.,* 2010, vol. 363, 711-723
- **KORN, E.L. ; LIU, P.-Y. ; LEE, S.J. ; CHAPMAN, J.-A.W. ; NIEDZWIECKI, D. ; SUMAN, V.J. ; MOON, J. ; SONDAK, V.K. ; ATKINS, M.B. ; EISENHAUER, E.A. et al.** Meta-analysis of phase II cooperative group trials in metastatic stage IV melanoma to determine progression-free and overall survival benchmarks for future phase II trials. *J. Clin. Oncol. Off. J. Am. Soc. Clin. Oncol,* 2008, vol. 26, 527-534
- **MANUEL, M. ; TREDAN, O. ; BACHELOT, T. ; CLAPISSON, G. ; COURTIER, A. ; PARMENTIER, G. ; RABEONY, T. ; GRIVES, A. ; PEREZ, S. ; MOURET, J.-F. et al.** Lymphopenia combined with low TCR diversity (divpenia) predicts poor overall survival in metastatic breast cancer patients. *Oncoimmunology,* 2012, vol. 1, 432-440
- **PAGES, F. ; BERGER, A. ; CAMUS, M. ; SANCHEZ-CABO, F. ; COSTES, A. ; MOLIDOR, R. ; MLECNIK, B. ; KIRILOVSKY, A. ; NILSSON, M. ; DAMOTTE, D. et al.** Effector memory T cells, early metastasis, and survival in colorectal cancer. *N. Engl. J. Med.,* 2005, vol. 353, 2654-2666
- **RIBAS, A.** Tumor Immunotherapy Directed at PD-1. *N. Engl. J. Med.,* 2012, vol. 366, 2517-2519